# EUROPÄISCHE PATENTSCHRIFT

(11) **EP 2 590 603 B1**
(45) Veröffentlichungstag und Bekanntmachung des Hinweises auf die Patenterteilung: **06.01.2016**
(21) Anmeldenummer: 11731275.1
(22) Anmeldetag: 08.07.2011
(51) Int. Cl.: A61F 5/01

(54) **VERBESSERTE KNIEBANDAGE MIT KORREKTURZÜGEL**
IMPROVED KNEE BANDAGE HAVING A CORRECTIVE STRAP
GENOUILLÈRE AMÉLIORÉE DOTÉE D'UNE BRIDE DE CORRECTION

(30) Priorität: 09.07.2010 DE 102010026680
(43) Veröffentlichungstag der Anmeldung: 15.05.2013
(73) Patentinhaber: Bauerfeind AG, 07937 Zeulenroda-Triebes (DE)
(72) Erfinder: SCHEUERMANN, Rainer, 24223 Raisdorf (DE); BAUERFEIND, Hans, B., 07937 Zeulenroda-Triebes (DE); HESS, Heinrich, 66271 Kleinblittersdorf (DE)
(74) Vertreter: Wohlfahrt, Jan Günther
(86) Internationale Anmeldenummer: PCT/EP2011/003408
(87) Internationale Veröffentlichungsnummer: WO 2012/003992

(56) Entgegenhaltungen:
- WO-A1-2005/087149
- DE-A1-102008 029 825
- DE-U1- 8 115 670
- FR-A1- 2 807 644
- US-A1- 2009 156 973

## Beschreibung

Die Erfindung betrifft eine verbesserte elastische Kniegelenkbandage, die Korrekturzügel zur Stabilisierung der Kniescheibe (Patella) aufweist. Gemäß der Erfindung ist der Korrekturzügel in seiner Wirkung auf die Kniescheibe einstellbar.

Die hier vorgestellte Kniegelenkbandage dient primär der Korrektur der Position der Kniescheibe durch gezielte einstellbare mechanische Krafteinwirkung. Aus elastischem Textilmaterial bestehende Kniegelenkbandagen zur Stabilisierung des Kniegelenks, die wie ein Strumpf über das Kniegelenk gezogen werden, sind bekannt. Häufig weisen solche Bandagen eine die Kniescheibe über eine Aussparung umfassende Profileinlage (Pelotte) auf, die an oder in dem Textilmaterial angeordnet ist. Die Profileinlage dient in Verbindung mit der durch die Eigenelastizität der Textilbandage auf das Kniegelenk erzeugten Kraft primär dazu, das Kniegelenk mechanisch zu stabilisieren, durch Entzündungsvorgänge entstandene Gewebswasseransammlungen zu drainieren und/oder die physiologisch korrekte Lage der Patella am Kniegelenk, je nach Beugungszustand des Knies, zu sichern. Dies dient der Therapie von Kniegelenksschäden. Die Bandage kann auch eingesetzt werden, weiteren Schädigungen des Kniegelenks vorzubeugen.

Zur Verbesserung der Funktion solcher Kniegelenkbandagen weisen diese zur Stabilisierung der Position der Patella mindestens ein biegsames, nicht dehnbares Spannglied in Form eines Korrekturzügels auf, das im Wesentlichen Bereiche der Kniescheibenpole wadenbeinseitig (Knieaußenseite, lateral) im Bogen um die Kniescheibe herum derart verbindet, dass bei Vergrößerung der Entfernung dieser Bereiche (Auseinanderwandern) im Zusammenhang mit dem Beugen des Kniegelenks der Abstand des Bogens des Korrekturzügels von der Patella verringert wird, sodass diese in Richtung medial zur Kniemitte hin verschoben oder gedrückt und/oder dort, in physiologisch korrekter Lage fixiert wird. Derartige Kniegelenkbandagen sind in der US 2009/0156973 A1, der WO 2005/087149 A1, der DE 10 2008 029 825 A1, der DE 38 38 576 A1 und DE 10 2004 040 793 A1 beschrieben.

Nachteilig bei solchen Kniegelenkbandagen ist besonders, dass die medial zentrierende Wirkung des Zügelbandes in vielen Anwendungsfällen unbefriedigend ist. In bestimmten Bewegungszuständen und/oder bei bestimmten zu therapierenden Schädigungen des Kniegelenks ist die Wirkung des Korrekturzügels auf die Patella zu gering ausgeprägt, sodass sich kein verwertbarer präventiver oder therapeutischer Effekt einstellt. Ebenso entsteht unter bestimmten anderen Bewegungsbedingungen, beispielsweise beim Sitzen mit gebeugtem Knie, häufig ein als zu stark empfundener und gegebenenfalls therapeutisch nachteiliger Druck auf die Patella. Es ist deshalb wünschenswert, eine Kniebandage der vorgenannten Art bereitzustellen, die diese Nachteile vermeidet. Der Erfindung liegt das technische Problem zugrunde, eine elastische Kniegelenkbandage bereitzustellen, worin die Verschiebungskraft, welcher ein in der Kniegelenkbandage angeordneter Korrekturzügel auf die Patella ausübt, an jeweils therapeutisch und prophylaktisch zweckmäßige Gegebenheiten angepasst werden kann, um den Therapieerfolg zu verbessern.

Die Erfindung löst das ihr zugrundeliegende technische Problem vollständig durch eine Bandage für ein Kniegelenk nach Anspruch 1, die einen elastischen Textilstrumpf und eine in oder an dem Textilstrumpf angeordnete ring- oder spangenförmige elastische Profileinlage, die zumindest den lateralen, das heißt wadenbeinseitigen, Abschnitt der Patella des Kniegelenks umgreift, sowie mindestens ein sich im Wesentlichen in Längsrichtung der Bandage über das Kniegelenk hinweg erstreckendes flexibles nicht dehnbares Zugband, das lateral in oder entlang der Profileinlage geführt ist, aufweist. Das Zugband weist ein oberes Ende und ein unteres Ende auf, wobei zumindest ein Ende über jeweils mindestens einen, oberen beziehungsweise unteren, Verankerungspunkt an dem Textilstrumpf lösbar, insbesondere verstellbar, verankert ist. In einem Abschnitt zwischen oberem Ende und unterem Ende des Zugbands kann dieses bei angelegter Bandage auf die Patella des Kniegelenks eine Kraft ausüben, die die Patella in ihre physiologische Normallage verschieben oder dort halten (fixieren) kann.

Wenn auf mindestens ein Ende des verankerten Zugbandes eine Zugkraft ausgeübt wird, die bei angelegter Bandage vor allem beim Beugen des Knies durch Auseinanderwandern der Verankerungspunkte erzeugt wird, wirkt diese dem Abschnitt als Kraftrektor verschiebend oder fixierend auf die Patella. Die fixierende Wirkung beruht darauf, dass, besonders bei einem Kniedefekt, einer im Gelenk auf die Patella wirkenden dislozierenden Kraft durch das Zugband eine entsprechend angepasste Gegenkraft entgegengesetzt wird, die der Verschiebung der Patella entgegenwirkt.

Die Erfindung zeichnet sich vor allem dadurch aus, dass die Wirklänge zwischen den Enden des Zugbands über eine nicht ortsfeste oder verstellbare Verankerung zumindest eines Endes des Zugbands veränderbar ist, um die von dem Zugband ausübbare, die Patella verschiebende oder fixierende Kraft zu steuern. Die Kraft, die das Zugband auf die Patella ausüben kann, hängt von dessen Wirklänge ab. Damit wird die effektive Länge des Bogens des Zugbands bezeichnet, die die Kraftausübung auf die Patella bei dem Auseinanderwandern der an den Verankerungspunkten verbundenen Enden beim Beugen des Knies bewirkt. Eine kurze Wirklänge entspricht einem im Gebrauch der Bandage gespannten Zugband, das eine stärkere Kraft ausüben kann; eine längere Wirklänge entspricht einem im Gebrauch weniger gespannten Zugband, das eine geringere Kraft auf die Patella ausübt.

Die Erfindung sieht also vor, das Zugband in seiner wirksamen Länge veränderbar auszuführen, um die verschiebende oder fixierende Wirkung des Zugbands und damit der Bandage auf die Patella jeweils nach Krankheitsbild, Therapieansatz, Bewegungszustand, Therapieverlauf, Therapieplan etc., insbesondere auch jeweils individuell für jeden Patienten, festlegen zu können.

In einer ersten Ausgestaltung der Bandage ist zumindest das obere Ende des Zugbands, das heißt das Ende, das zur Körpermitte hinweist, wahlweise an einem von mehreren beabstandeten Verankerungspunkten örtlich veränderbar fixiert. Beispielsweise sind mehrere primär in Verlaufsrichtung des Zugbands hintereinander liegende Verankerungspunkte mit dem Zugbandende alternativ lösbar verbindbar, um so die Wirklänge des Zugbandes zu steuern. In einer weiteren Variante dieser Ausgestaltung ist alternativ ausschließlich das untere Ende des Zugbandes entsprechend ortsveränderlich fixierbar. In einer weiteren Variante sind beide Enden des Zugbandes entsprechend ortsveränderlich fixierbar.

Bevorzugt ist die ortsveränderliche Fixierung über eine lösbare Verbindung realisiert, die ausgewählt ist aus Klettverbindungen, Verknopfungen und Haken/Öse-Systemen. Die lösbare Verbindung ist aber nicht auf diese Strukturen beschränkt. Der Fachmann kennt weitere lösbare Verbindungen, die besonders auch im Bereich von Textilbandagen ohne Weiteres einsetzbar sind.

Es kann vorgesehen sein, die alternativen Verankerungspunkte zur veränderbaren Fixierung des Zugbandes farblich oder über Ziffern oder Symbole zu markieren, sodass der Patient ohne Weiteres nach vorheriger Anweisung durch den Orthopäden oder Orthopädietechniker die Verstellung der Wirklänge des Zugbandes gegebenenfalls einfach und zuverlässig selbst vornehmen kann, beispielsweise in Abhängigkeit vom Therapieerfordernis, festgelegten Therapieplan und/oder vom aktuellen Bewegungszustand des Patienten.

In einer anderen Variante davon wird die ortsveränderliche Fixierbarkeit des Zugbandes durch mindestens einen an oder im Bereich des Verankerungspunktes vorgesehenen Aufrollmechanismus realisiert, um die Wirklänge einzustellen. Ein solcher Aufrollmechanismus kann zusätzlich mit einem an sich bekannten gegen einen Widerstand rastbaren Stoppmechanismus versehen sein. In einer weiteren alternativen Ausgestaltung ist am Verankerungspunkt ein an sich bekannter Flaschenzugmechanismus vorgesehen, um zusätzlich die Verstellbarkeit zu verbessern, besonders den Kraftaufwand bei der Verstellung zu vereinfachen.

Die Erfindung sieht vor, dass das Zugband zwar flexibel, aber im Wesentlichen unelastisch ist. Unelastisch im Sinne der vorliegenden Erfindung bedeutet primär, dass der Elastizitätsmodul des Zugbandmaterials und/oder des inneren Aufbaus des Zugbands im Vergleich zu den weiteren bei der Bandage verwendeten elastischen Materialien, besonders dem Textilgewebe und der Profileinlage, deutlich größer ist, das heißt besonders um eine oder mehrere Zehnerpotenzen. Während bevorzugte Materialien für die Profileinlage beispielsweise Silikonelastomere mit einer mittleren Shore A-Härte sind, beispielsweise also Shore A 30 bis 60, ist das Zugband beispielsweise in Form eines Drahts, Seils oder Geflechts aus steifem Material wie Stahl oder Nylon gefertigt. Der Fachmann kennt andere damit vergleichbare Materialien und Strukturen, die im Sinne der Erfindung ebenso zur Ausgestaltung als erfindungsgemäß einsetzbares Zugband geeignet sind; die Erfindung umfasst auch solche weiteren Materialien und Strukturen.

In einer alternativen, erfindungsgemäßen Ausgestaltung wird das Zugband in einem an oder in der Profileinlage angeordneten widerstandsfähigen Gleitkanal geführt. Dieser kann fest mit der Profileinlage verbunden sein und ist insbesondere so ausgestaltet, dass die durch das Zugband ausgeübten Seitenkräfte während der Benutzung der Bandage nicht unmittelbar auf die Profileinlage wirken können, wodurch diese zerstört werden könnte, besonders da dort bei der Benutzung reibende, messerschneidende Kräfte zwischen Zugband und Polstermaterial der Profileinlage auftreten. Bevorzugt ist der Gleitkanal in Form einer Röhre oder Rinne ausgebildet, und zwar aus einem mechanisch zähen und abriebfesten Werkstoff, beispielsweise Teflon, Nylon, der ein reibungsarmes Gleiten des Zugbands unter Vermeidung von Materialabrieb ermöglicht.

In einer weiteren Ausgestaltung ist der Führungskanal selbst ebenfalls elastisch. Dabei ist das Elastizitätsmodul des Materials des Führungskanals so gewählt, dass dieses mit dem Elastizitätsmodul der Profileinlage harmoniert, sodass besonders Schwerkräfte vermieden werden, welche die Verbindung zwischen Führungskanal und Profileinlage aufbrechen.

In einer alternativen Ausgestaltung ist das Zugband ortsfest innerhalb oder an der Profileinlage fixiert. So können reibende und schneidende Krafteinwirkungen auf das Polstermaterial verhindert werden; das Polstermaterial folgt an der Grenzfläche zwischen Zugband und Profileinlage der Bewegung des Zugbands. Beispielsweise kann das Zugband in das Material der Profileinlage eingegossen sein. Alternativ kann das Zugband auf die Profileinlage oder in einer alternativen, erfindungsgemäßen Ausführungsform auf den Polsterbezug der Profileinlage aufgeklebt oder aufgeschweißt sein. Insbesondere ist das Zugband so angeordnet, dass es im angelegten Zustand zwischen Profileinlage und dem Knie verläuft, um im Wesentlichen unmittelbar in Kontakt zu der zu korrigierenden oder fixierenden Patella zu stehen. Dazu kann erfindungsgemäß das Zugband direkt an oder innerhalb des zwischen Profileinlage und Patella angeordneten Polsterbezugs verlaufen. In einer alternativen, erfindungsgemäßen Ausführungsform ist das Zugband mit diesem vernäht oder verschweißt. Alternativ oder zusätzlich ist das Zugband selbst unmittelbar mit dämmendem Material umhüllt, um eine mechanische Reizung der Haut zu vermeiden. Dies kann dann zweckmäßig sein, wenn alternativ das Zugband außerhalb der Profileinlage, vorzugsweise direkt am Kniegelenk entlang verläuft. Beispielsweise ist das Zugband aus silikonisiertem, das heißt mit Silikonelastomer umhüllten Nylongarn ausgestaltet, das ein mit Silikonelastomer umhülltes Gewebeband bildet.

In einer weiteren, zusätzlichen oder alternativen, Ausgestaltung ist das Zugband an der Bandage auswechselbar ausgeführt. Es kann für den Einsatz bei ein und derselben Bandage in alternativen Dicken, Längen und/oder Elastizitäten, ausgebildet sein. So kann es beispielsweise erforderlich sein, je nach Bewegungszustand in ein und dieselbe Bandage einmal ein steiferes und ein anderes Mal ein elastischeres Zugband einzusetzen. So kann die Wirkung des Zugbandes zusätzlich zur Verstellbarkeit der Zugbandenden über einen größeren Bereich eingestellt werden, um durch die Anpassbarkeit die Funktion der Kniegelenkbandage weiter zu verbessern.

Erfindungsgemäß ist vorgesehen, dass das Zugband im Bereich der Patella lateral an der Innenseite, das heißt der dem Knie zugewandten Innenfläche, der Bandage verläuft, und an zumindest einem Durchtrittspunkt der Bandage aus der Innenseite des Textilstrumpfs austritt und auf der Außenseite, das heißt auf der dem Knie abgewandten Außenfläche der Bandage verläuft, um dort, bevorzugt am Textilstrumpf selbst, an zumindest einem Verankerungspunkt verankert zu sein. Dadurch wird vorteilhafterweise das Verstellen der Wirklänge auch während des Tragens der Bandage ermöglicht. Außerdem kann so vermieden werden, dass die Mittel, welche die erfindungsgemäße lösbare ortsvariable Verbindung gestalten, beispielsweise Verknopfungen, nicht an der Innenseite der Bandage, das heißt zum Körper hin, angeordnet zu sein brauchen, was dort nachteiligerweise mit lokaler mechanischer Reizung der Haut verbunden sein kann. Die Offenbarung ist aber nicht auf außen liegende lösbare Verbindungsmittel oder ein Außenverlauf zumindest eines Endes des Zugbands beschränkt.

Besonders ist vorgesehen, dass ein Ende des Zugbandes unmittelbar an oder in dem Textilstrumpf nicht lösbar oder verstellbar ortsfest fixiert ist, während das andere Ende erfindungsgemäß verstellbar fixierbar ist. Dazu ist das Zugband vorzugsweise mit dem Textilstrumpf vernäht, verklebt oder verschweißt. In einer bevorzugten Ausgestaltung ist das untere Ende des Zugbandes mit dem Textilstrumpf verschweißt oder verklebt, und das obere Ende des Zugbandes ist von der Innenseite an die Außenseite der Bandage geführt, wo es, für den Träger leicht erreichbar, an seinem Ende verstellbar fixiert werden kann.

In einer Ausgestaltung der Bandage weist diese an oder in dem Textilstrumpf auf der Seite der Bandage mindestens ein sich in Längsausdehnung der Bandage erstreckendes stabilisierendes Federstabelement auf, um die Kniegelenkbandage als solche mechanisch zu stabilisieren. In einer besonderen Ausgestaltung dieser Ausführung ist nun vorgesehen, dass mindestens einer der Verankerungspunkte am Ende des Zugbandes unmittelbar mit dem Federstabelement in kraftschlüssiger Verbindung steht. Auf diese Weise kann eine verbesserte Ortsfixierung des Zugbandendes erreicht werden, was die Kraftausübung auf das Zugband durch das Beugen des Knies und damit die mechanische Wirkung auf die Patella verbessert.

Die Bandage hilft, durch Fehlstellung der Kniescheibe bedingte Schmerzen zu lindern. Vorteilhafterweise kann auch der Verschleiß des hinter der Kniescheibe sitzenden Kniegelenkknorpels verhindert werden. Gegenstand der Erfindung ist auch die medizinische, therapeutische und/oder prophylaktische Verwendung der erfindungsgemäßen Bandage zur Prophylaxe, Linderung und/oder Therapie von Erkrankungen des Gelenkapparats des tierischen oder menschlichen Körpers, besonders Erkrankungen des Kniegelenks, die mit einer unphysiologischen Verschiebung der Patella verbunden sind. Besonders sind dies die Patellalateralisation, die Patelladysplasie, die Retropatellaarthrose, die Chondropathia patellae, das Patellaspitzensyndrom, die Chondromalacia patellae, der Zustand nach Patella-Luxation sowie posttraumatische und postoperative Zustände.

Die Erfindung wird durch die Figuren und zugehörigen Beschreibungen näher beschrieben, ohne dass diese beschränkend zu verstehen wären.

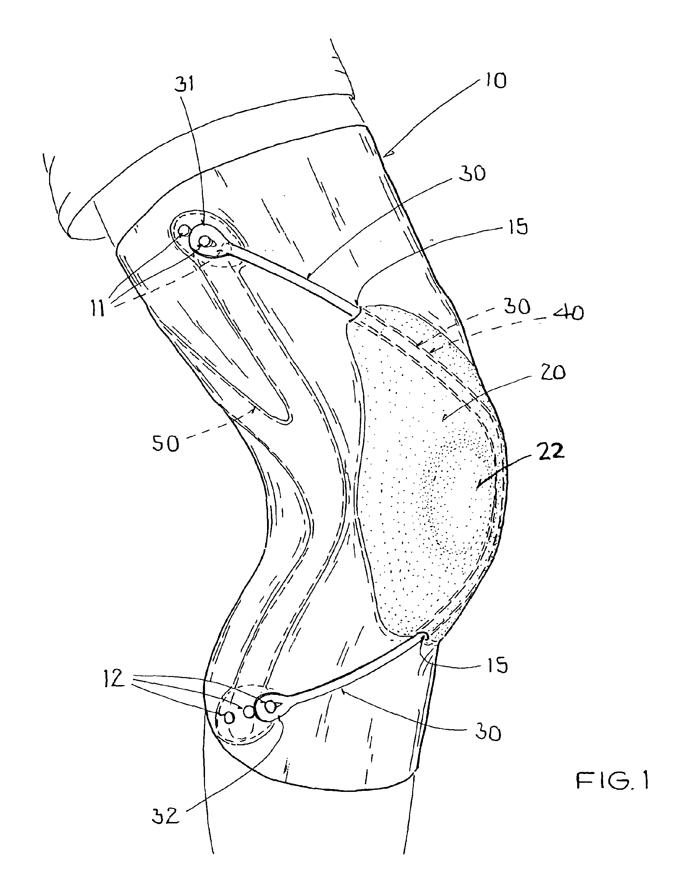

Figur 1 zeigt eine Seitenansicht auf eine besondere Ausführungsform der erfindungsgemäßen Kniegelenkbandage in schematischer Darstellung. Dargestellt ist eine Draufsicht auf die Innenseite des Kniegelenks. An einem Textilstrumpf 10 ist zur Innenseite, das heißt zum Kniegelenk hin, eine die Patella umgreifende Profileinlage 20 angeordnet. Die Profileinlage zentriert sich an einer Fensterung oder Materialverdünnung 22 über der Patella. Ein zumindest im Bereich der Profileinlage 20, innerhalb des Textilstrumpfes 10 verlaufendes im Wesentlichen unelastisches flexibles Zugband 30 wird entlang der Profileinlage in einem Kanal 40 geführt. Das Zugband 30 tritt durch die Durchtrittsöffnung 15 von der Innenseite des Textilgewebes auf die Außenseite, um dort jeweils über das obere Ende 31 oder das untere Ende 32 an variablen Verankerungspunkten 11, 12 fixiert zu werden. In einer besonderen Ausgestaltung weist der Textilstrumpf zusätzlich ein Federstabelement 50 auf. Die Verankerungspunkte 11, 12 können mit dem Federstabelement 50 in kraftschlüssiger Verbindung stehen, um die Verankerungspunkte mechanisch zu stabilisieren.

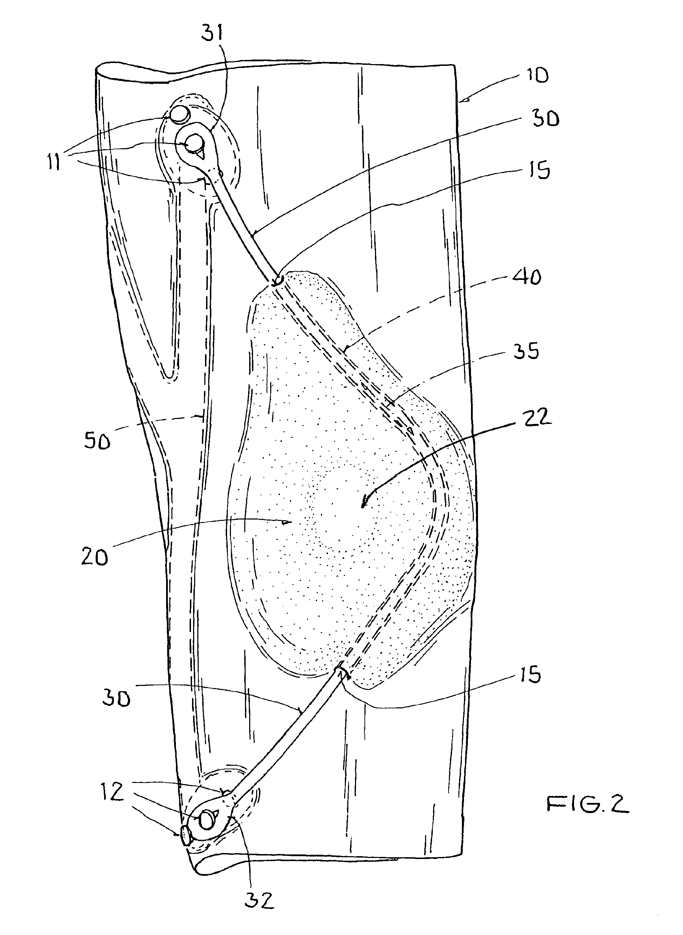

Figur 2 zeigt eine Draufsicht der abgelegten Bandage nach Figur 1. Das Zugband 30 wirkt über die Wirklänge 35 verschiebend oder fixierend auf die Patella, die in angelegter Bandage im Bereich der Fensterung/Aussparung 22 der Profileinlage 20 zu liegen kommt.

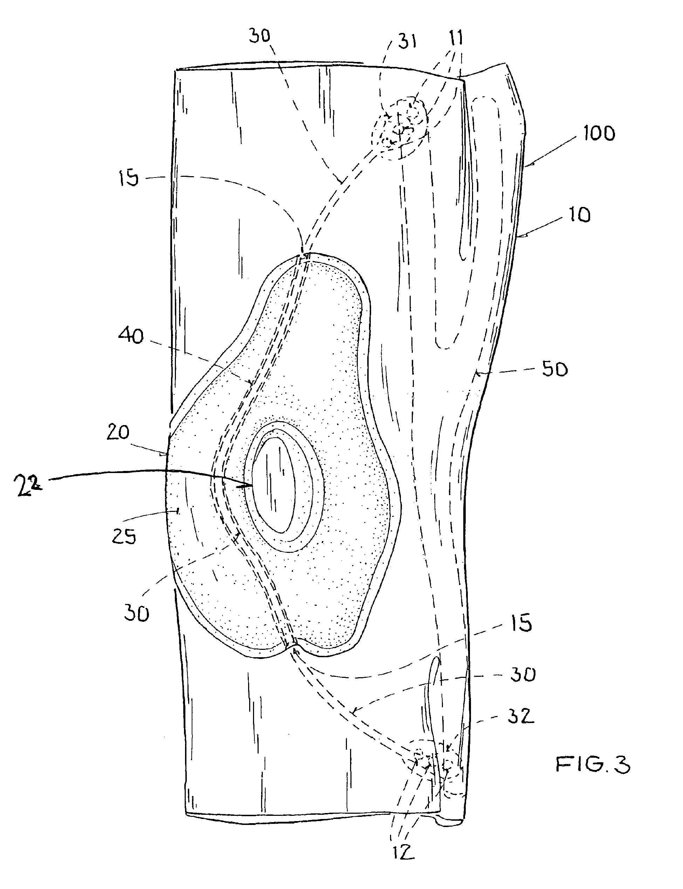

Figur 3 zeigt eine Innenansicht der abgelegten Bandage nach Figuren 1 und 2. Die Profileinlage 20 ist über den Polsterbezug 25, der am äußeren umlaufenden Rand und im Bereich der Fensterung/Aussparung 22 mit dem Textilmaterial des Textilstrumpfs 2 verschweißt oder verklebt ist, mit dem Textilstrumpf verbunden. Innerhalb der Profileinlage wird das Zugband 30 in dem Gleitkanal 40 geführt. An den Durchtrittsöffnungen 15 tritt das Zugband jeweils auf die Außenseite des Textilstrumpfes.

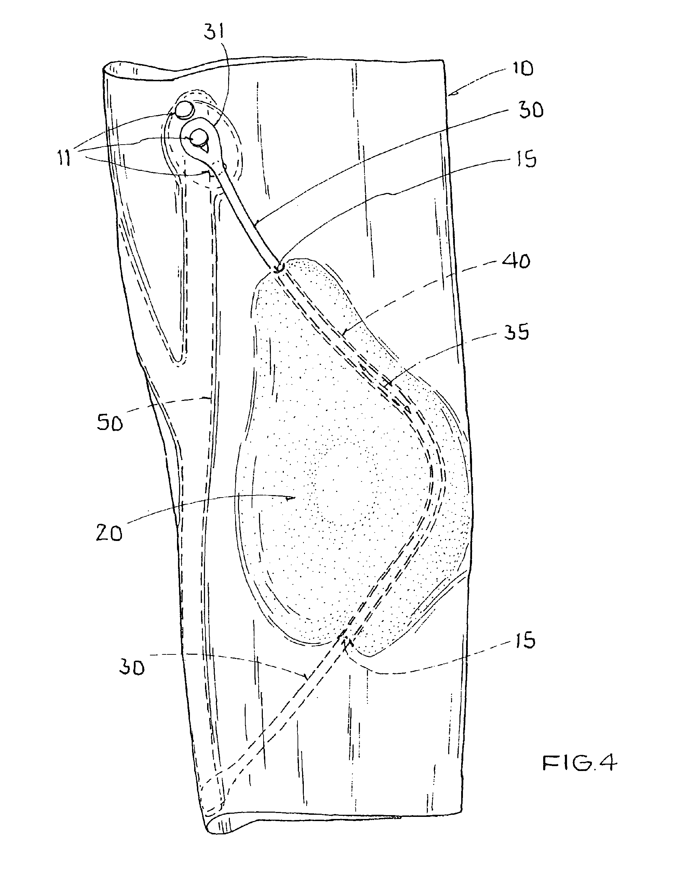

Figur 4 zeigt eine alternative Ausgestaltung der erfindungsgemäßen Bandage. Das Zugband 30 ist im Bereich des unteren Endes an oder innerhalb des Textilbandes ortsfest fixiert. Eine Verstellmöglichkeit ergibt sich über das obere Ende 31 in Verbindung mit den variablen Verknüpfungspunkten 11. Eine Durchtrittsöffnung 15 am unteren Abschnitt des Zugbandes kann entfallen, wenn das Zugband an oder in der Innenseite des Textilstrumpfs 10 fixiert wird.

## Patentansprüche

1. Bandage (100) für Kniegelenk mit elastischem Textilstrumpf (10), einer in oder an dem Textilstrumpf (10) angeordneten ring- oder spangenförmigen elastischen Profileinlage (20) zum Umgreifen zumindest des lateralen, wadenbeinseitigen Abschnitts der Patella des Kniegelenks und mindestens einem sich im Wesentlichen in Längsrichtung der Bandage über das Kniegelenk hinweg erstreckenden flexiblen nicht dehnbaren Zugband (30), das lateral in oder entlang der Profileinlage (20) geführt ist und über eine Wirklänge (35) des Zugbands zwischen einem oberen Ende (31) und einem unteren Ende (32), die über jeweils mindestens einen oberen und unteren Verankerungspunkt (11,12) an dem Textilstrumpf (10) verankert sind, bei angelegter Bandage beim Beugen des Knies durch Auseinanderwandern der Verankerungspunkte (11,12) eine die Patella zentrierende Kraft ausübbar ist, wobei die Wirklänge (35) des Zugbands an zumindest einem Ende (31,32) an dem Verankerungspunkt (11,12) veränderbar ist, um die von dem Zugband (30) ausübbare, die Patella zentrierende Kraft zu steuern, wobei das Zugband (30) an zumindest einem Durchtrittspunkt (15) aus der Innenseite des Textilstrumpfs (10) an dessen Außenseite tritt, wobei das Zugband (30) zumindest im Bereich der Patella innerhalb des Textilstrumpfs (10) und in oder an der zur Patella hin weisenden Seite der Profileinlage (20) verläuft, wobei das Zugband (30) an einem oder innerhalb eines zwischen Profileinlage (20) und Patella angeordneten Polsterbezugs (25) verläuft und wobei das Zugband (30) mit dem Polsterbezug (25) verschweißt, verklebt oder vernäht ist oder in einem an oder in der Profileinlage (20) angeordneten Gleitkanal (40) geführt ist.

2. Bandage nach Anspruch 1, wobei die Wirklänge (35) des Zugbands durch an dem Verankerungspunkt (11,12) vorgesehenen Aufrollmechanismus veränderbar ist.

3. Bandage nach Anspruch 1, wobei die Wirklänge (35) des Zugbands durch eine am Verankerungspunkt (11,12) vorgesehene verstellbare lösbare Verbindung veränderbar ist.

4. Bandage nach Anspruch 3, wobei die lösbare Verbindung ausgewählt ist aus: Klettverbindung, Verknopfung und Haken/Öse-System.

5. Bandage nach einem der vorstehenden Ansprüche, wobei an der in dem Textilstrumpf (10) auf der Seite der Bandage mindestens ein sich in Längsausdehnung der Bandage erstreckendes stabilisierendes Federstabelement (50) angeordnet ist und zumindest ein Verankerungspunkt (11,12) des Zugbands (30) mit dem Federstabelement (50) in kraftschlüssiger Verbindung steht.

6. Bandage nach einem der vorstehenden Ansprüche, wobei das Zugband (30) eine damit fest verbundene polsternde Beschichtung (32) aufweist.

7. Bandage nach Anspruch 6, wobei die polsternde Beschichtung (32) ein Überzug im Silikonelastomer ist.

## Claims

1. A bandage (100) for a knee joint, comprising an elastic textile stocking (10), an annular or clasp-like elastic profiled insert (20) arranged in or at the textile stocking (10) to encompass at least the lateral fibula-side section of the patella of the knee joint, and at least one tensile strap (30) which is flexible but not elastic and essentially extending in the longitudinal direction of the bandage across the knee joint and which is guided laterally in or along the profiled insert (20), wherein, when the bandage is worn, via an effective length (35) of the tensile strap between an upper end (31) and a lower end (32), each of which ends are anchored to the textile stocking (10) through at least one upper and one lower anchoring point (11, 12), a force that is centering the patella can be exerted due to the anchoring points (11, 12) being moved apart when the knee is bent, wherein the effective length (35) of the tensile strap can be modified at at least one end (31, 32) at the anchoring point (11, 12) in order to control the force that is centering the patella and that is exerted by the tensile strap (30), wherein the tensile strap (30) exits from the inside of the textile stocking (10) to the outside thereof at at least one passage point (15), wherein, at least in the vicinity of the patella, the tensile strap (30) extends inside the textile stocking (10) and in or on the side of the profiled insert (20) facing the patella, wherein the tensile strap (30) extends at or inside a pad cover coat (25) that is arranged between the profiled insert (20) and the patella, and wherein the tensile strap (30) is sealed, glued or sewed to the pad cover coat (25) or is guided in a slide channel (40) that is arranged at or in the profiled insert (20).

2. The bandage according to claim 1, wherein the effective length (35) of the tensile strap can be changed by means of a coiling mechanism provided at the anchoring point (11, 12).

3. The bandage according to claim 1, wherein the effective length (35) of the tensile strap can be changed by an adjustable severable connection provided at the anchoring point (11, 12).

4. The bandage according to claim 3, wherein the severable connection is selected from the group consisting of hook and pile connection, buttoned connection, and hook and eye system.

5. The bandage according to any one of the preceding claims, wherein at least one stabilizing spring bar element (50) that extends in the longitudinal direction of the bandage is arranged in the textile stocking (10) on the side of the bandage, and at least one anchoring point (11, 12) of the tensile strap (30) is connected to the spring bar element (50) in a force-fitted manner.

6. The bandage according to any one of the preceding claims, wherein the tensile strap (30) comprises a padding covering (32) that is firmly connected thereto.

7. The bandage according to claim 6, wherein the padding covering (32) is a cover coat in the silicone elastomer.

## Revendications

1. Bandage (100) pour articulation du genou comprenant un bas textile élastique (10), un insert profilé (20) élastique en forme de bague ou d'agrafe disposé dans ou sur le bas textile (10) pour entourer au moins le tronçon latéral côté péroné de la rotule de l'articulation du genou, et au moins une bande de traction (30) flexible ne pouvant se dilater, s'étendant par-dessus l'articulation du genou essentiellement dans le sens longitudinal du bandage, qui est dirigée latéralement dans ou le long de l'insert profilé (20) et, par une longueur active (35) de la bande de traction entre une extrémité supérieure (31) et une extrémité inférieure (32) qui sont ancrées sur le bas textile (10) par respectivement au moins des points d'ancrage supérieur et inférieur (11, 12), une force centrant la rotule peut être exercée lorsque le bandage est posé, lorsque le genou est fléchi, par un éloignement des points d'ancrage (11, 12), sachant que la longueur active (35) de la bande de traction est modifiable sur au moins une extrémité (31, 32) sur le point d'ancrage (11, 12) afin de commander la force centrant la rotule pouvant être exercée par la bande de traction (30), sachant que la bande de traction (30) sort sur le côté extérieur de la face intérieure du bas textile (10) en au moins un point de passage (15), sachant que la bande de traction (30) s'étend, au moins dans la région de la rotule, dans le bas textile (10) et dans l'insert profilé (20) ou sur le côté de l'insert profilé orienté vers la rotule, sachant que la bande de traction (30) passe sur ou à l'intérieur d'une enveloppe de garnissage (25) disposée entre l'insert profilé (20) et la rotule, et sachant que la bande de traction (30) est soudée, collée ou cousue à l'enveloppe de garnissage (25) ou est dirigée dans un canal de glissement (40) disposé sur ou dans l'insert profilé (20).

2. Bandage selon la revendication 1, dans lequel la longueur active (35) de la bande de traction est modifiable par un mécanisme de déroulement prévu sur le point d'ancrage (11, 12).

3. Bandage selon la revendication 1, dans lequel la longueur active (35) de la bande de traction est modifiable par une liaison séparable réglable prévue sur le point d'ancrage (11, 12).

4. Bandage selon la revendication 3, dans lequel la liaison séparable est sélectionnée parmi : une liaison par velcro, un boutonnage ou un système de crochets/oeillet.

5. Bandage selon l'une des revendications précédentes, dans lequel, dans le bas textile (10), sur le côté du bandage, au moins un élément de tige à ressort (50) stabilisant s'étirant dans l'allongement longitudinal du bandage est disposé et au moins un point d'ancrage (11, 12) de la bande de traction (30) est en liaison par force avec l'élément de tige à ressort (50).

6. Bandage selon l'une des revendications précédentes, dans lequel la bande de traction (30) présente un revêtement de garnissage (32) fixé avec celle-ci.

7. Bandage selon la revendication 6, dans lequel le revêtement de garnissage (32) est un revêtement en élastomère de silicone.
